# EUROPEAN PATENT SPECIFICATION

(11) **EP 2 874 690 B1**
(45) Date of publication and mention of the grant of the patent: **24.03.2021**
(21) Application number: 13742136.8
(22) Date of filing: 17.07.2013
(51) Int. Cl.: A61M 25/01, A61M 25/04, A61M 25/00

(54) **GUIDE EXTENSION CATHETER**
FÜHRUNGSVERLÄNGERUNGSKATHETER
CATHÉTER D'EXTENSION DE GUIDAGE

(30) Priority: 17.07.2012 US 201261672664 P
(43) Date of publication of application: 27.05.2015
(73) Proprietor: Boston Scientific Scimed, Inc., Maple Grove, MN 55311 (US)
(72) Inventor: WANG, Huisun, Maple Grove, Minnesota 55311 (US); WASDYKE, Joel M., Eden Prairie, MN 55347 (US); FALK, Wayne, Minneapolis, Minnesota 55406 (US); BLIX, John, Maple Grove, Minnesota 55311 (US)
(74) Representative: Vossius & Partner Patentanwälte Rechtsanwälte mbB
(86) International application number: PCT/US2013/050930
(87) International publication number: WO 2014/015062

(56) References cited:
- EP-A1- 1 639 951
- EP-A2- 0 710 490
- WO-A2-01/41858
- US-A1- 2009 005 757
- US-A1- 2010 234 876

## Description

### Technical Field

The present disclosure pertains to medical devices. More particularly, the present disclosure pertains to elongated intracorporeal medical devices including a guide extension catheter.

### Background

A wide variety of intracorporeal medical devices have been developed for medical use, for example, intravascular use. Some of these devices include guidewires, catheters, and the like. These devices are manufactured by any one of a variety of different manufacturing methods and may be used according to any one of a variety of methods. Of the known medical devices and methods, each has certain advantages and disadvantages. There is an ongoing need to provide alternative medical devices as well as alternative methods for manufacturing and using medical devices.

EP 0 710 490 A2 describes a balloon catheter with improved pressure source including an elongate shaft with an inflatable balloon connected to its distal end and a manifold connected to its proximal end. The manifold may be fixedly or removably attached to the shaft and includes a barrel with a plunger movably disposed inside it.

WO 01/41858 A2 relates to a catheter suitable for drug delivery which comprises a catheter body comprising a proximal and a distal end, and defining a drug delivery lumen and a stylet lumen. The stylet lumen comprises a distal end configured to permit a stylet to abut the stylet lumen distal end, and a proximal aperture which is distal the proximal end of the catheter, providing entry of the stylet into the side of the catheter. The stylet lumen is adapted for slidably receiving a stylet which can be used to guide the catheter to the intended site in the body of a subject.

US 2010/0234876 A1 relates to an apparatus for recapturing an ablation balloon including a movable collar and a push wire. The movable collar comprises a proximal attachment region and a distal capture region sized to capture an expandable body therein. The push wire is configured to couple to the attachment region.

EP 1 639 951 A1 relates to an intravascular foreign matter suction assembly that is insertable into a blood vessel having a relatively small diameter and exhibits a high suction force. The intravascular foreign matter suction assembly includes a combination of a guiding catheter for being inserted to an ostium of a coronary artery of the aorta and a suction catheter inserted in the lumen of the guiding catheter and extending farther than the distal end of the guiding catheter for removing a foreign matter in a blood vessel which exists at a target location in the coronary artery. The suction catheter includes a tubular portion provided on the distal end side and a wire portion provided on the proximal end side for the tubular portion and wherein the wire portion has a distal end embedded in a wall which forms the tubular portion. The assembly further comprises a distal end protective catheter and a guide wire. The distal end of the distal end protective catheter is inserted in the lumen of the suction catheter and the guide wire is inserted in the lumen of the distal end protective catheter. The distal end protective catheter includes a distal end tubular portion and a proximal end side wire-like portion formed from a metal wire.

US 2009/005757 A1 relates to a catheter apparatus that has a flexible shaft, guidewire lumens passing through the shaft, and a positioning device for positioning the guidewires relative to an external lumen. The positioning device may be an expansible scaffold covered with a retractable sheath. The positioning device may also be one or more balloons that are inflated through an inflation port in the shaft. The catheter apparatus is operated by inserting a guide catheter into a vasculature with a chronic total occlusion, inserting the catheter apparatus into the guide catheter, and advancing the guidewires through the guidewire lumens and into contact with a chronic total occlusion. The positioning device is activated before and/or between attempts to pass the guidewires through the chronic total occlusion to interrogate various parts of the chronic total occlusion.

### Brief Summary

The present invention relates to a guide extension catheter as defined by the claims. The guide extension catheter comprises a proximal member having a proximal outer diameter. The proximal member takes the form of a tether wire. A removable stiffening member is disposed adjacent to the proximal member. The stiffening member includes a tube. A distal sheath member is attached to the proximal member. The distal sheath member has a distal outer diameter greater than the proximal outer diameter.

An example guide extension catheter system is also described. The guide extension catheter system may include a guide catheter having an inner diameter and a guide extension catheter extending through the guide catheter. The guide extension catheter may include a proximal shaft, a removable stiffening member coupled to the proximal shaft, and a distal sheath member attached to the proximal shaft. The distal sheath member may have an outer diameter that is configured to substantially fit within the inner diameter of the guide catheter.

Methods for accessing a coronary artery are also described. An example method may include providing a guide catheter and advancing the guide catheter through a blood vessel to a position adjacent to an ostium of a coronary artery. The method may also include providing a guide extension catheter. The guide extension catheter may include a proximal member having a proximal outer diameter. A removable stiffening member may be disposed adjacent to the proximal member. A distal sheath member may be attached to the proximal member. The distal sheath member may have a distal outer diameter greater than the proximal outer diameter. The method may also include advancing the guide extension catheter through the guide catheter to a position where at least a portion of the distal sheath extends distally beyond a distal end of the guide catheter and into the coronary artery, removing the stiffening member from the proximal member, and advancing a treatment catheter through the guide catheter.

The above summary of some examples is not intended to describe each disclosed example or every implementation of the present invention. The Figures, and Detailed Description, which follow, more particularly exemplify these examples.

### Brief Description of the Drawings

The invention may be more completely understood in consideration of the following detailed description of various examples of the invention in connection with the accompanying drawings, in which:
Figure 1 is a plan view illustrating an example guide catheter advanced through the aorta to the ostium of a coronary artery;
Figure 2 is a plan view illustrating an example guide extension catheter used in conjunction with a guide catheter;
Figure 3 is a cross-sectional side view of an example guide extension catheter;
Figure 4 is a cross-sectional side view of the example guide extension catheter and an example guide catheter;
Figure 5 is a partial cross-sectional view of an example guide extension catheter;
Figure 6 is a partial cross-sectional view of the example guide extension catheter illustrated in Figure 5 in another configuration;
Figure 7 is a partial cross-sectional view of the example guide extension catheter illustrated in Figure 5 in another configuration;
Figure 8 is a partial cross-sectional view of a portion of a guide extension catheter of the invention;
Figure 9 is a partial cross-sectional view of the guide extension catheter illustrated in Figure 8 with a removable stiffening member removed;
Figure 10 is a partial cross-sectional view of a portion of another embodiment of the guide extension catheter;
Figure 11 is a partial cross-sectional view of a portion of another embodiment of the guide extension catheter;
Figure 12 is a side view of an example catheter system including an organizing member;
Figure 13 is an end view of an example organizing member; and
Figure 14 is an end view of another example organizing member.

### Detailed Description

For the following defined terms, these definitions shall be applied, unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

The following detailed description should be read with reference to the drawings in which similar elements in different drawings are numbered the same. The drawings, which are not necessarily to scale, depict illustrative examples and embodiments and are not intended to limit the scope of the invention.

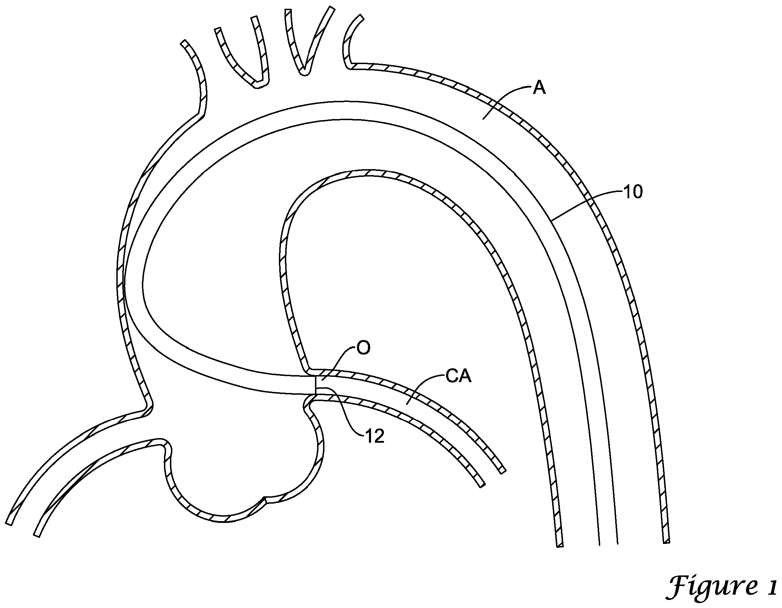

Minimally-invasive cardiac interventions such as percutaneous transluminal coronary angioplasty are widely utilized throughout the world. These procedures may include the use of a guide catheter. For example, a guide catheter 10 may be advanced through a blood vessel such as the aorta A to a position adjacent to the ostium O of a (e.g., left and/or right) coronary artery CA as illustrated in Figure 1. When so positioned, a treatment catheter (e.g., balloon catheter, stent delivery system, etc.) may be advanced through guide catheter 10 and into the coronary artery CA to a target location where the treatment catheter may be used to perform the appropriate cardiac intervention.

In order for the treatment catheter to efficiently reach the intended target location, maintaining the position of guide catheter 10 at the ostium O of the coronary artery CA may be desirable. For example, given that the heart may be beating during the intervention (and/or other factors), the guide catheter 10 may lose its positioning or otherwise be shifted so that it no longer is positioned to efficiently guide the treatment catheter to the coronary arteries. This may include a distal end 12 of guide catheter 10 being shifted away from the ostium O of the coronary artery CA. Because of the shift away from the ostium O, access to the coronary arteries CA may require repositioning of guide catheter 10 in order to bring the distal end 12 back into engagement with the ostium O of the coronary artery CA.

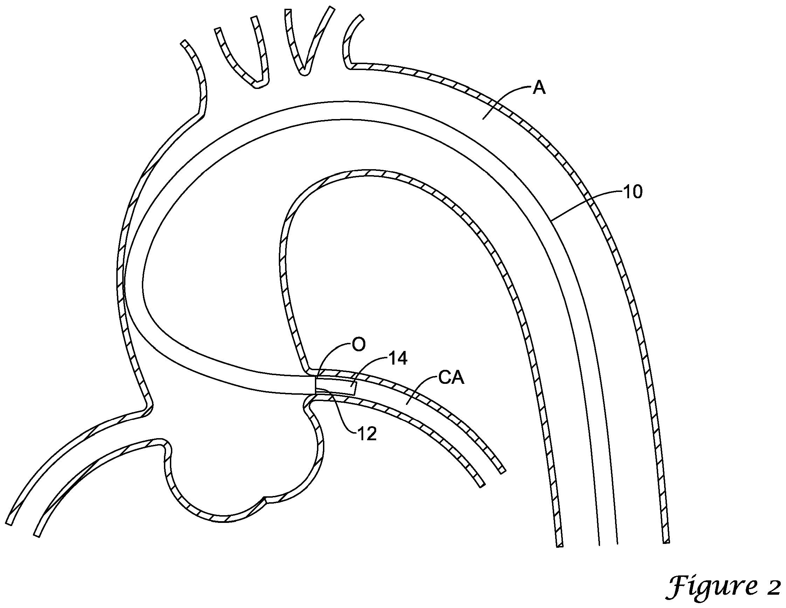

Disclosed herein are medical devices, in particular a guide extension catheter, that may improve access to the coronary arteries CA. For example, Figure 2 illustrates a guide extension catheter 14 extending through guide catheter 10 and beyond distal end 12 of guide catheter 10 into the coronary artery CA. Because, for example, guide extension catheter 14 may extend beyond distal end 12 of guide catheter 10, guide extension catheter 14 may extend beyond the ostium O of the coronary artery CA and into a portion of the coronary artery CA. By extending beyond the ostium O, the guide extension catheter 14 may stabilize the positioning of guide catheter 10 and allow for improved access to the coronary artery CA for a number of cardiac interventions.

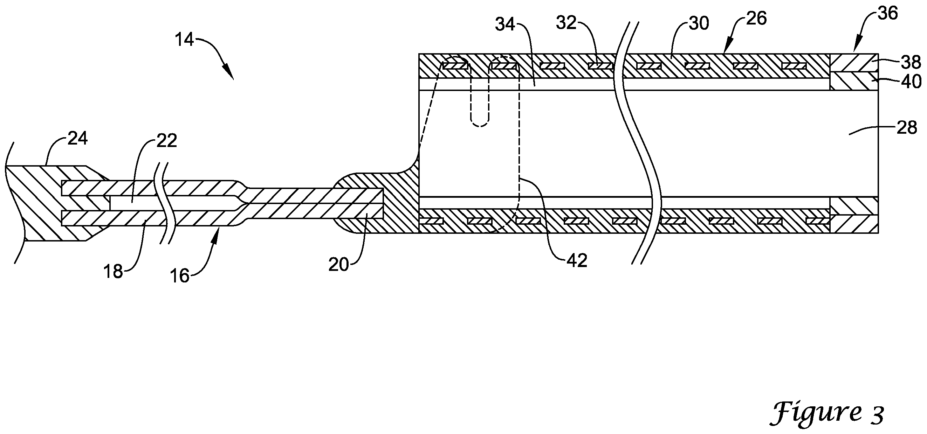

Figure 3 is a cross-sectional side view of guide extension catheter 14. Here it can be seen that guide extension catheter 14 may include a proximal shaft or member 16. Proximal member 16 may include a proximal portion 18 and a distal or ribbon portion 20. Proximal portion 18 may have a lumen 22 defined therein. In some examples, lumen 22 extends along the entire length of proximal portion 18. In other examples, lumen 22 extends along only a portion of the length of proximal portion 18. In addition, proximal portion 18 may include both proximal and distal openings (e.g., positioned at the proximal and distal end of proximal portion 18) such that lumen 22 is "open" on both ends. Alternatively, one or both of the ends of proximal portion 18 may be closed or otherwise sealed. For example, the distal end of proximal portion 18 may be closed. In some of these and in other examples, proximal portion 18 may have an opening or port (not shown) formed in the wall of proximal portion 18 and spaced from the proximal and/or distal end of proximal portion 18. The port may or may not be in fluid communication with lumen 22. A hub 24 may be attached to proximal portion 18.

A distal sheath 26 may be attached to proximal member 16. Sheath 26 may have a lumen 28 formed therein. In general, lumen 28 (and/or the inner diameter of distal sheath 26) may be larger than lumen 22 (and/or the inner diameter of proximal portion 18) and may be larger than the outer diameter of proximal member 16. Accordingly, lumen 28 may be sufficiently large so as to allow a therapeutic catheter (e.g., balloon catheter, stent delivery system, etc.) to pass therethrough. For example, when guide extension catheter 14 is positioned within guide catheter 10, the therapeutic catheter may extend within guide catheter 10 alongside proximal member 16 and through lumen 28 of distal sheath 26.

Distal sheath 26 may include a body portion 30. In at least some examples, body portion 30 may include one or more polymers including any of those disclosed herein. This may include the use of polymers with a differing durometer along the length of body portion 30. For example, a more proximal section of body portion 30 may include a polymer with a higher durometer and a more distal section of body portion 30 may include a polymer with a lower durometer. Portions of all of the length of body portion may be loaded with or otherwise include a radiopaque material. Body portion 30 may also include a reinforcement member 32. The form of reinforcement member 32 may vary. For example, reinforcement member 32 may include a braid, coil, mesh, or the like.

An inner liner or layer 34 may be disposed along an inner surface of body portion 30. The form of liner 34 may vary. For example, liner 34 may be a lubricious liner or otherwise include a lubricious material such as polytetrafluoroethylene. A tip member 36 may be attached body portion 30, for example at a distal end of body portion 30. In some examples, tip member 36 may be a single layer of material. Alternatively, tip member may include an outer layer 38 and an inner layer 40. Outer layer 38 and inner layer 40 may be formed from the same material. In some of these examples, outer layer 38 and inner layer 40 may include the same polymeric material and each be loaded with the same or different radiopaque materials. For example, inner layer 40 may include a polyether block amide loaded with approximately 75-95% (e.g., about 90%) by weight tungsten and outer layer 38 may include a polyether block amide loaded with approximately 30-50% (e.g., 40%) by weight bismuth subcarbonate. These are just example. In other examples, outer layer 38 and inner layer 40 may be made from different materials.

Distal sheath 26 may be attached to ribbon portion 20 of proximal member 16. The arrangement and/or configuration of the attachment between ribbon portion 20 and distal sheath 26 may vary. For example, distal sheath 26 may have an opening or lumen formed in tube wall thereof and ribbon portion 20 may be disposed within the opening. This may include necking, skiving, or pinching down ribbon portion 20 and inserting the necked down portion into the opening. In some examples, inserting ribbon portion 20 into the opening may secure proximal member 16 to distal sheath 26 via a mechanical bond. In some of these and in other examples, additional and/or alternative bonding may be utilized including those bonding mechanisms commonly used for medical devices (e.g., adhesive bonding, welding, thermal bonding, brazing, etc.). Other attachment mechanisms are also contemplated for attaching proximal member 16 to distal sheath 26 including direct bonding (e.g., adhesive bonding, thermal bonding, welding, brazing, etc.), bonding that is facilitated by a third component such as a metal or polymer collar 42 that may be bonded between the ribbon portion 20 and distal sheath 26.

Guide extension catheter 14 may also include a number of coatings that may, for example, reduce friction. For example, proximal member 16 may have an inner and/or outer coating that includes a hydrophilic polymer that may reduce friction during tracking. An example coating may include BAYER CL-100, BIOSLIDE, NG-HPC, SLIP COAT, MDX, or the like. These are just examples. Other materials are contemplated including those disclosed herein.

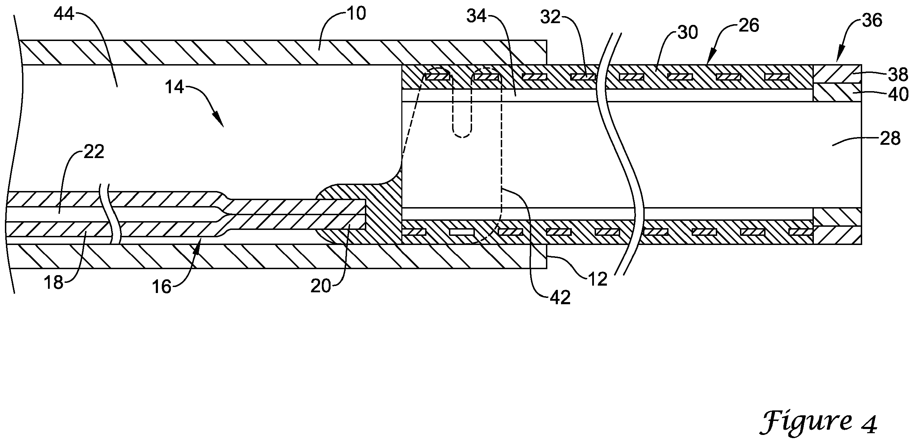

Figure 4 illustrates guide extension catheter 14 disposed within guide catheter 10 (e.g., disposed within a lumen 44 defined within guide catheter 10). As shown, distal sheath 26 may be arranged to extend distally out from distal end 12 of guide catheter 10. When so arranged, distal sheath 26 may engage the ostium O and/or extend within a portion of the coronary artery CA to help maintain the position of guide catheter 10 and improve access to the coronary artery CA. Proximal member 16 may be designed to be sufficiently small (while still being sufficiently sized and configured for pushability) so as to take up relatively little space within the interior or lumen 44 of guide catheter 10. Accordingly, the use of guide extension catheter 14 allows for a therapeutic catheter or medical device to be advanced through guide catheter 10 in order to reach the desired target location for the intervention. In some examples, proximal member 16 may contact the inner wall surface of guide catheter 10, which may provide even more space.

When designing guide extension catheter, it may be desirable to incorporate structures that may provide structural "push" support. However, the use of such structures may add bulk and/or stiffness to the guide extension catheter. Disclosed herein are guide extension catheters that include pushing structures and/or stiffening members. The stiffening members may be removable from the guide extension catheter such that additional push support and/or stiffness can be added/removed as needed during an intervention. The use of a removable stiffening member may provide additional space within the guide catheter for other therapeutic devices to pass therethrough, upon removal of the stiffening member.

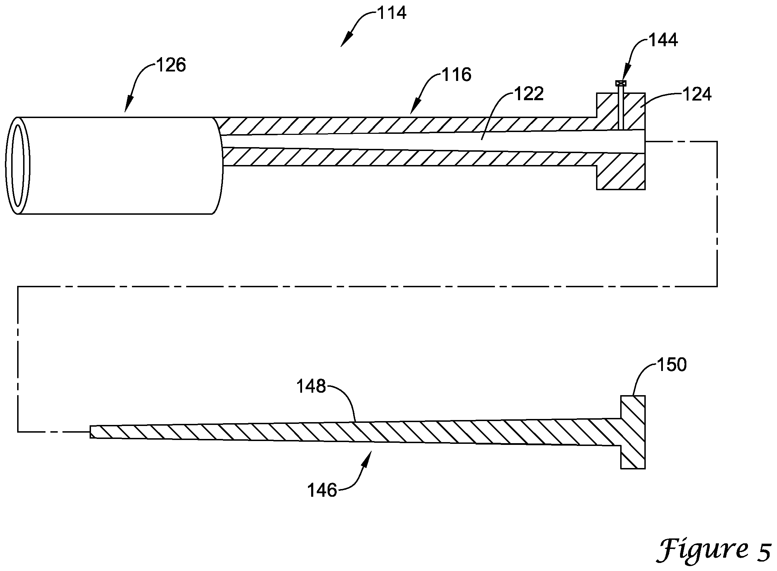

Figure 5 illustrate an example guide extension catheter 114 that may be similar in form and function to other guide extension catheters disclosed herein. Guide extension catheter 114 may include proximal member 116 and distal sheath 126. The structures are shown schematically. It can be appreciated that the form and/or structural configuration of proximal member 116 and/or distal sheath 126 may resemble other proximal members and distal sheaths (e.g., proximal member 16 and distal sheath 26) disclosed herein.

In at least some examples, proximal member 116 may be tubular in form and defines a lumen 122. Lumen 122 may have a constant diameter or lumen 122 may have a changing or tapered diameter as shown. Proximal member 116 may also include a proximal or handle region 124. A locking member 144 may be coupled to proximal member 116, for example at or adjacent to proximal region 124. The precise form of locking member 144 may vary. In at least some examples, locking member 144 may include a set screw. Alternatively, locking member 144 may include a clamp, vice, collet, or the like or any other suitable locking structure.

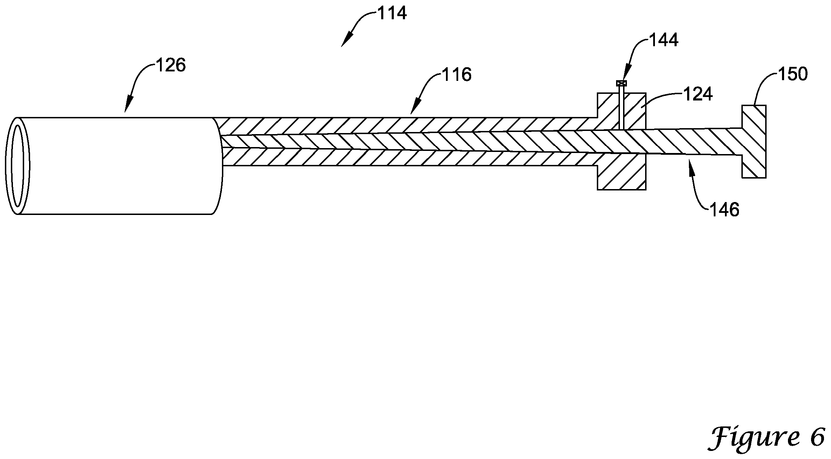

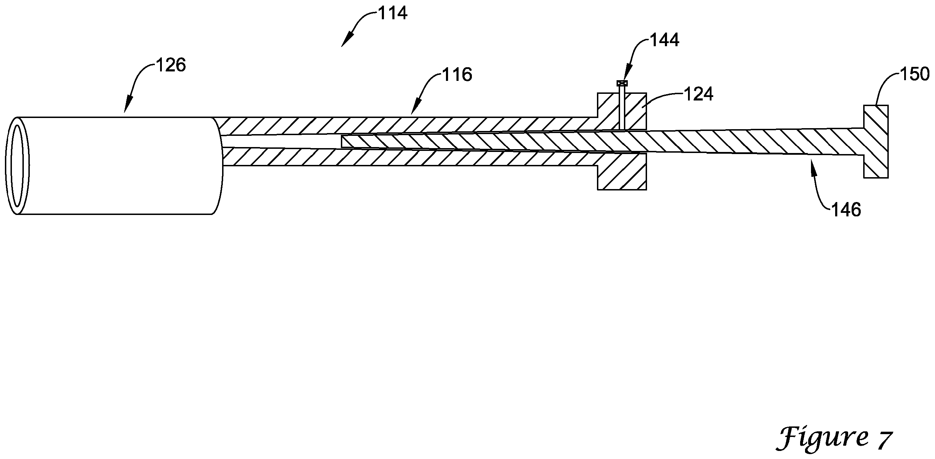

Guide extension catheter 114 may also include a removable stiffening member 146. Stiffening member 146 may include a tapered body portion 148 and a proximal or handle region 150. The shape, form, and/or configuration of body portion 148 can vary. For example, body portion 148 can have a constant outer diameter, one or more steps in diameter, or other variations including variations in flexibility (e.g., more flexible along distal portions and more stiff along proximal portions). In addition, body portion 148 can have a circular cross-sectional shape or in other examples, may have a non-circular cross-sectional shape. Stiffening member 146 may be configured to be disposed within lumen 122 of proximal member 116 as shown in Figure 6. This may add stiffening or "push" support to guide extension catheter 114. Stiffening member 146 may be extended with lumen 122 to essentially any suitable position. For example stiffening member 146 may be extended substantially to the distal end of proximal member 116 (e.g., as shown in Figure 6) or any portion of the length of proximal member 116 including partially through lumen 122 as illustrated in Figure 7.

In use, guide extension catheter 114 may be advanced through guide catheter 10. While advancing guide extension catheter 114, stiffening member 146 may be inserted into lumen 122 (e.g., partially or fully) so as to provide the desired stiffness and/or pushability to guide extension catheter 114. While advancing guide extension catheter 114, stiffening member 146 can be longitudinally shifted within lumen 122, as desired, to alter the stiffness of guide extension catheter 114 in a manner that best suit the needs of the intervention. When guide extension catheter 114 is properly position within guide catheter 10, stiffening member 146 may be removed from lumen 122.

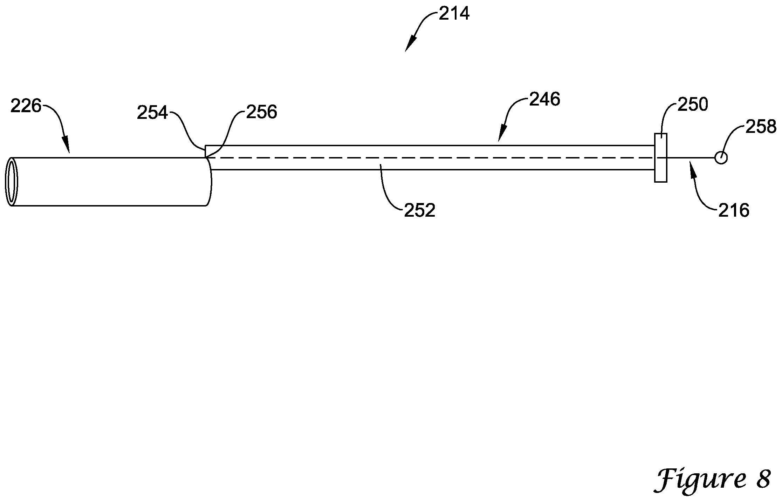

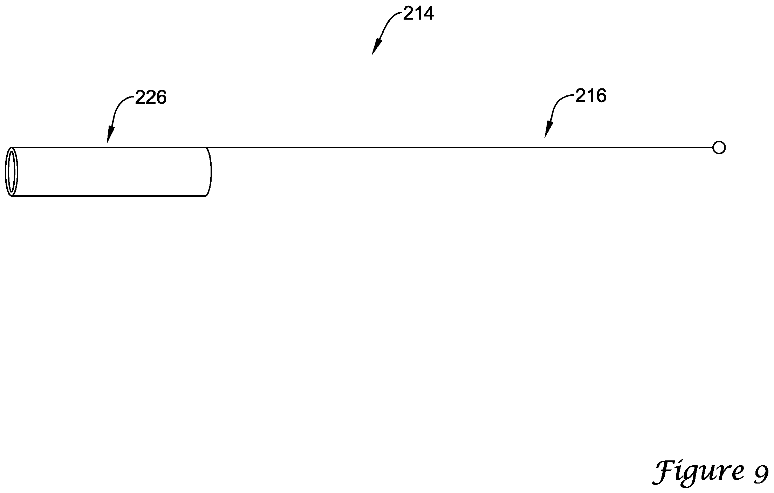

Figure 8 illustrates a first embodiment of a guide extension catheter according to the invention. The guide extension catheter 214 that may be similar in form and function as other guide extension catheters described herein. Guide extension catheter 214 includes proximal member 216 and distal sheath 226. Guide extension catheter 214 includes stiffening member 246. In at least some examples, stiffening member 246 may include proximal or handle region 250 and a lumen 252. A distal end 256 of stiffening member 246 may be free from attachment to distal sheath 226. Alternatively, distal end 256 of stiffening member 246 may be attached to distal sheath 226 in a manner that permits removal at an appropriate time during the intervention. In general, distal end 256 of stiffening member may be configured to engage distal sheath 226. Accordingly, stiffening member 246 may be used to provide stiffening support or otherwise "push" distal sheath 226 to the desired position during delivery of guide extension catheter 214. When properly positioned, stiffening member 246 may be proximally retracted or removed from proximal member 216 as shown in Figure 9.

Proximal member 216 may extend through lumen 252. According to the invention, proximal member 216 takes the form of a tether wire. For example, proximal member 216 may include a cable having a reduced outer diameter so as to reduce the amount of space proximal member 216 may take up within guide catheter 10. In at least some examples, proximal member 216 may have sufficient strength so that a user can pull on proximal member 216 to proximally retract guide extension catheter 214. Proximal member 216 may not have sufficient rigidity so that proximal member 216 may be used to "push" distal sheath 226 and, instead, stiffening member 246 provides additional pushing capabilities to guide extension catheter 214. Proximal member 216 has a distal end 256 that is attached to distal sheath 226 and a proximal or handle region 258.

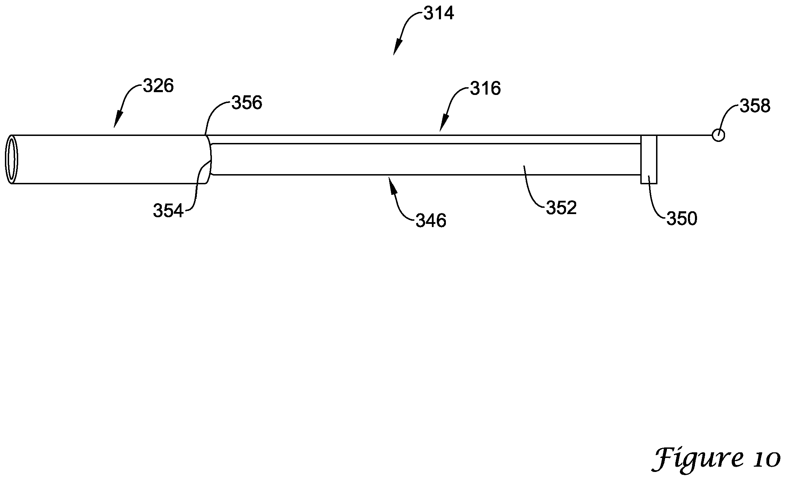

Figure 10 illustrates a second embodiment of the guide extension catheter 314 that may be similar in form and function as other guide extension catheters disclosed herein. Guide extension catheter 314 includes proximal member 316 and distal sheath 326. Guide extension catheter 314 includes stiffening member 346. In at least some examples, stiffening member 346 may include proximal or handle region 350 and a lumen 352. Proximal member 316 may extend through lumen 352 or, as shown in Figure 10, proximal member 316 may extend along the outer wall of stiffening member 346. Proximal member 316 takes the form of tether wire. Proximal member 316 has a distal end 356 that is attached to distal sheath 326 and a proximal or handle region 358.

A distal end 356 of stiffening member 346, which includes a tube may engage distal sheath 326. Accordingly, stiffening member 346 may be used to provide stiffening support or otherwise "push" distal sheath 326 to the desired position. When properly positioned, stiffening member 346 may be proximally retracted or removed from proximal member 316.

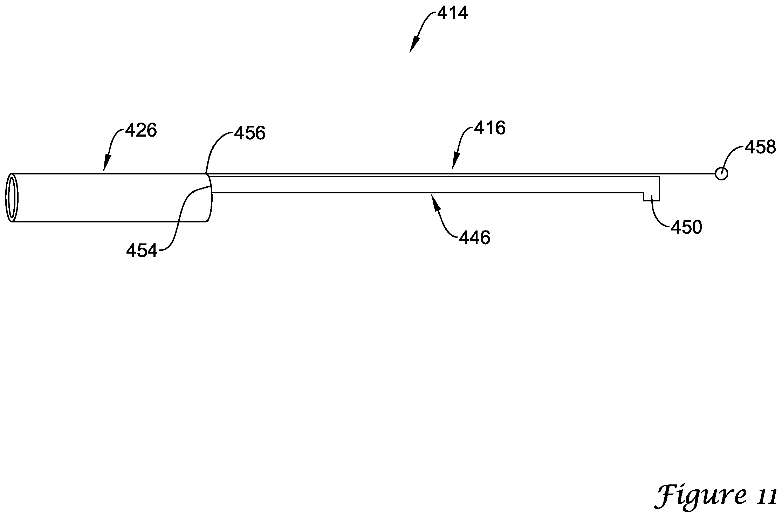

Figure 11 illustrates third embodiment of guide extension catheter 414 that may be similar in form and function as other guide extension catheters disclosed herein. Guide extension catheter 414 includes proximal member 416 and distal sheath 426. Guide extension catheter 414 includes stiffening member 446. In at least some examples, stiffening member 446 includes a tube and may include a proximal or handle region 450. Proximal member 416 may extend along an outer surface of stiffening member. Proximal member 416 takes the form of a tether wire. Proximal member 416 has a distal end 456 that is attached to distal sheath 426 and a proximal or handle region 458.

A distal end 456 of stiffening member 446, which includes a tube may engage distal sheath 426. Accordingly, stiffening member 446 may be used to provide stiffening support or otherwise "push" distal sheath 426 to the desired position. When properly positioned, stiffening member 446 may be proximally retracted or removed from proximal member 416.

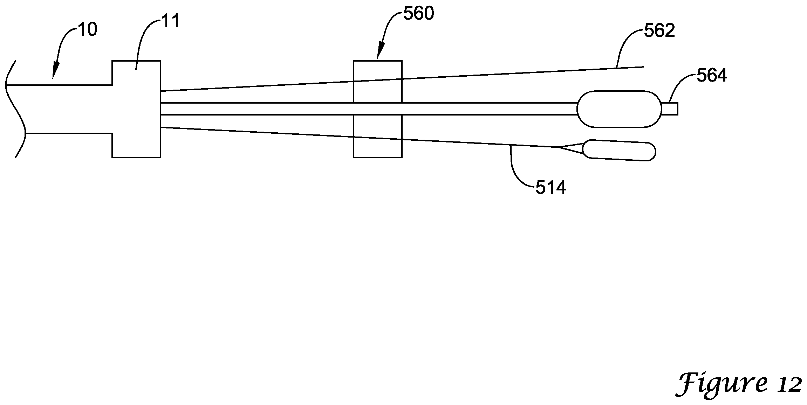

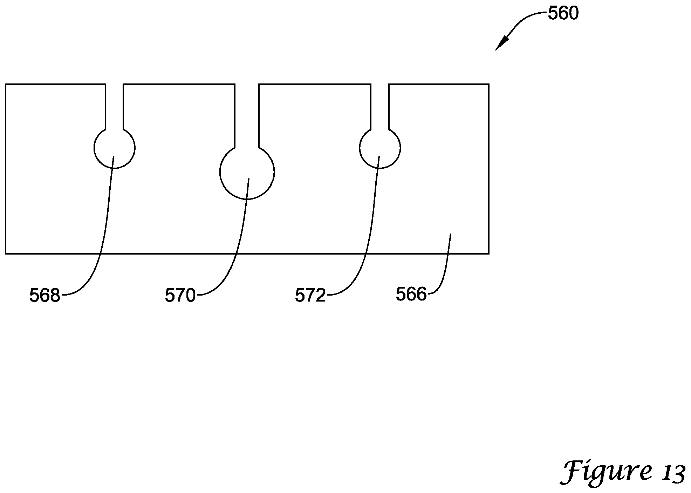

Figure 12 illustrates an example organizer or organizing member 560 that may be utilized with any of the guide extension catheters disclosed herein. Organizing member 560 may aid a clinician using guide catheter 10 (e.g., which may have a hub 11 formed thereon) manage a plurality of devices. For example, some interventions may employ the use of a guidewire 562, a therapeutic catheter 564 (e.g., a balloon catheter, a stent delivery system, or the like), and a guide extension catheter 514 (and/or different or other devices). Organizing member 560 may be configured to engage and hold these devices. For example, organizing member 560 may include a body 566 having a plurality of openings formed therein as shown in Figure 13. For example, body 566 may include a first opening 568, a second opening 570, and a third opening 572. While no particular arrangement may be necessary, openings 568/570/572 may be utilized to house guidewire 562, catheter 564, and/or guide extension catheter 514.

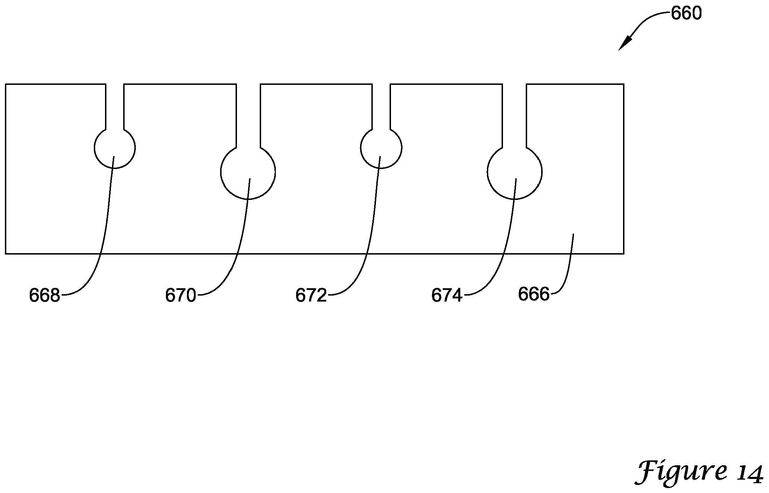

The size, shape, number, and configuration of openings 568/570/572 may vary. For example, another example organizing member 660 is shown in Figure 14. Organizing member 660 may include body 666 with first opening 668, second opening 670, third opening 672, and a fourth opening 674. Such an organizing member 660 may be configured to house guidewire 562, catheter 564, and guide extension catheter 514 as well as separately house a stiffening member such as any of those disclosed herein. Other organizing members are also contemplated that utilized openings with smaller, larger, wider, or differently shaped openings.

The materials that can be used for the various components of the guide extension catheters disclosed herein may vary. For simplicity purposes, the following discussion makes reference to proximal member 16 and distal sheath 26. However, this is not intended to limit the devices and methods described herein, as the discussion may be applied to other similar tubular members and/or components of tubular members or devices disclosed herein.

Proximal member 16 and distal sheath 26 and/or other components of guide extension catheter 14 may be made from a metal, metal alloy, polymer (some examples of which are disclosed below), a metal-polymer composite, ceramics, combinations thereof, and the like, or other suitable material. Some examples of suitable metals and metal alloys include stainless steel, such as 304V, 304L, and 316LV stainless steel; mild steel; nickel-titanium alloy such as linear-elastic and/or super-elastic nitinol; other nickel alloys such as nickel-chromium-molybdenum alloys (e.g., UNS: N06625 such as INCONEL® 625, UNS: N06022 such as HASTELLOY® C-22®, UNS: N10276 such as HASTELLOY® C276®, other HASTELLOY® alloys, and the like), nickel-copper alloys (e.g., UNS: N04400 such as MONEL® 400, NICKELVAC® 400, NICORROS® 400, and the like), nickel-cobalt-chromium-molybdenum alloys (e.g., UNS: R30035 such as MP35-N® and the like), nickel-molybdenum alloys (e.g., UNS: N10665 such as HASTELLOY® ALLOY B2®), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R30003 such as ELGILOY®, PHYNOX®, and the like); platinum enriched stainless steel; titanium; combinations thereof; and the like; or any other suitable material.

As alluded to herein, within the family of commercially available nickel-titanium or nitinol alloys, is a category designated "linear elastic" or "non-super-elastic" which, although may be similar in chemistry to conventional shape memory and super elastic varieties, may exhibit distinct and useful mechanical properties. Linear elastic and/or non-super-elastic nitinol may be distinguished from super elastic nitinol in that the linear elastic and/or non-super-elastic nitinol does not display a substantial "superelastic plateau" or "flag region" in its stress/strain curve like super elastic nitinol does. Instead, in the linear elastic and/or non-super-elastic nitinol, as recoverable strain increases, the stress continues to increase in a substantially linear, or a somewhat, but not necessarily entirely linear relationship until plastic deformation begins or at least in a relationship that is more linear that the super elastic plateau and/or flag region that may be seen with super elastic nitinol. Thus, for the purposes of this disclosure linear elastic and/or non-super-elastic nitinol may also be termed "substantially" linear elastic and/or non-super-elastic nitinol.

In some cases, linear elastic and/or non-super-elastic nitinol may also be distinguishable from super elastic nitinol in that linear elastic and/or non-super-elastic nitinol may accept up to about 2-5% strain while remaining substantially elastic (e.g., before plastically deforming) whereas super elastic nitinol may accept up to about 8% strain before plastically deforming. Both of these materials can be distinguished from other linear elastic materials such as stainless steel (that can also can be distinguished based on its composition), which may accept only about 0.2 to 0.44 percent strain before plastically deforming.

In some examples, the linear elastic and/or non-super-elastic nickel-titanium alloy is an alloy that does not show any martensite/austenite phase changes that are detectable by differential scanning calorimetry (DSC) and dynamic metal thermal analysis (DMTA) analysis over a large temperature range. For example, in some examples, there may be no martensite/austenite phase changes detectable by DSC and DMTA analysis in the range of about -60 degrees Celsius (°C) to about 120 °C in the linear elastic and/or non-super-elastic nickel-titanium alloy. The mechanical bending properties of such material may therefore be generally inert to the effect of temperature over this very broad range of temperature. In some examples, the mechanical bending properties of the linear elastic and/or non-super-elastic nickel-titanium alloy at ambient or room temperature are substantially the same as the mechanical properties at body temperature, for example, in that they do not display a super-elastic plateau and/or flag region. In other words, across a broad temperature range, the linear elastic and/or non-super-elastic nickel-titanium alloy maintains its linear elastic and/or non-super-elastic characteristics and/or properties.

In some examples, the linear elastic and/or non-super-elastic nickel-titanium alloy may be in the range of about 50 to about 60 weight percent nickel, with the remainder being essentially titanium. In some examples, the composition is in the range of about 54 to about 57 weight percent nickel. One example of a suitable nickel-titanium alloy is FHP-NT alloy commercially available from Furukawa Techno Material Co. of Kanagawa, Japan. Some examples of nickel titanium alloys are disclosed in U.S. Patent Nos. 5,238,004 6,508,803. Other suitable materials may include ULTANIUM™ (available from Neo-Metrics) and GUM METAL™ (available from Toyota). In some other examples, a superelastic alloy, for example a superelastic nitinol can be used to achieve desired properties.

In at least some examples, portions or all of proximal member 16 and/or distal sheath 26 may also be loaded with, made of, or otherwise include a radiopaque material. Radiopaque materials are understood to be materials capable of producing a relatively bright image on a fluoroscopy screen or another imaging technique during a medical procedure. This relatively bright image aids the user of guide extension catheter 14 in determining its location. Some examples of radiopaque materials can include, but are not limited to, gold, platinum, palladium, tantalum, tungsten alloy, polymer material loaded with a radiopaque filler (e.g., barium sulfate, bismuth subcarbonate, etc.), and the like. Additionally, other radiopaque marker bands and/or coils may also be incorporated into the design of guide extension catheter 14 to achieve the same result.

In some examples, a degree of Magnetic Resonance Imaging (MRI) compatibility is imparted into guide extension catheter 14. For example, proximal member 16 and distal sheath 26, or portions thereof, may be made of a material that does not substantially distort the image and create substantial artifacts (i.e., gaps in the image). Certain ferromagnetic materials, for example, may not be suitable because they may create artifacts in an MRI image. Proximal member 16 and distal sheath 26, or portions thereof, may also be made from a material that the MRI machine can image. Some materials that exhibit these characteristics include, for example, tungsten, cobalt-chromium-molybdenum alloys (e.g., UNS: R30003 such as ELGILOY®, PHYNOX®, and the like), nickel-cobalt-chromium-molybdenum alloys (e.g., UNS: R30035 such as MP35-N® and the like), nitinol, and the like, and others.

A sheath or covering (not shown) may be disposed over portions or all of proximal member 16 and distal sheath 26 that may define a generally smooth outer surface for guide extension catheter 14. In other examples, however, such a sheath or covering may be absent from a portion of all of guide extension catheter 14, such that proximal member 16 and distal sheath 26 may form the outer surface. The sheath may be made from a polymer or other suitable material. Some examples of suitable polymers may include polytetrafluoroethylene (PTFE), ethylene tetrafluoroethylene (ETFE), fluorinated ethylene propylene (FEP), polyoxymethylene (POM, for example, DELRIN® available from DuPont), polyether block ester, polyurethane (for example, Polyurethane 85A), polypropylene (PP), polyvinylchloride (PVC), polyether-ester (for example, ARNITEL® available from DSM Engineering Plastics), ether or ester based copolymers (for example, butylene/poly(alkylene ether) phthalate and/or other polyester elastomers such as HYTREL® available from DuPont), polyamide (for example, DURETHAN® available from Bayer or CRISTAMID® available from Elf Atochem), elastomeric polyamides, block polyamide/ethers, polyether block amide (PEBA, for example available under the trade name PEBAX®), ethylene vinyl acetate copolymers (EVA), silicones, polyethylene (PE), Marlex high-density polyethylene, Marlex low-density polyethylene, linear low density polyethylene (for example REXELL®), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR®), polysulfone, nylon, nylon-12 (such as GRILAMID® available from EMS American Grilon), perfluoro(propyl vinyl ether) (PFA), ethylene vinyl alcohol, polyolefin, polystyrene, epoxy, polyvinylidene chloride (PVdC), poly(styrene-b-isobutylene-b-styrene) (for example, SIBS and/or SIBS 50A), polycarbonates, ionomers, biocompatible polymers, other suitable materials, or mixtures, combinations, copolymers thereof, polymer/metal composites, and the like. In some examples the sheath can be blended with a liquid crystal polymer (LCP). For example, the mixture can contain up to about 6 percent LCP.

In some examples, the exterior surface of the guide extension catheter 14 (including, for example, the exterior surface of proximal member 16 and distal sheath 26) may be sandblasted, beadblasted, sodium bicarbonate-blasted, electropolished, etc. In these as well as in some other examples, a coating, for example a lubricious, a hydrophilic, a protective, or other type of coating may be applied over portions or all of the sheath, or in examples without a sheath over portion of proximal member 16 and distal sheath 26, or other portions of guide extension catheter 14. Alternatively, the sheath may comprise a lubricious, hydrophilic, protective, or other type of coating. Hydrophobic coatings such as fluoropolymers provide a dry lubricity which improves guidewire handling and device exchanges. Lubricious coatings improve steerability and improve lesion crossing capability. Suitable lubricious polymers are well known in the art and may include silicone and the like, hydrophilic polymers such as high-density polyethylene (HDPE), polytetrafluoroethylene (PTFE), polyarylene oxides, polyvinylpyrolidones, polyvinylalcohols, hydroxy alkyl cellulosics, algins, saccharides, caprolactones, and the like, and mixtures and combinations thereof. Hydrophilic polymers may be blended among themselves or with formulated amounts of water insoluble compounds (including some polymers) to yield coatings with suitable lubricity, bonding, and solubility. Some other examples of such coatings and materials and methods used to create such coatings can be found in U.S. Patent Nos. 6,139,510 and 5,772,609.

The coating and/or sheath may be formed, for example, by coating, extrusion, co-extrusion, interrupted layer co-extrusion (ILC), or fusing several segments end-to-end. The layer may have a uniform stiffness or a gradual reduction in stiffness from the proximal end to the distal end thereof. The gradual reduction in stiffness may be continuous as by ILC or may be stepped as by fusing together separate extruded tubular segments. The outer layer may be impregnated with a radiopaque filler material to facilitate radiographic visualization. Those skilled in the art will recognize that these materials can vary widely without deviating from the scope of the present invention.

It should be understood that this disclosure is, in many respects, only illustrative. Changes may be made in details, particularly in matters of shape and size. The invention's scope is, of course, defined in the language in which the appended claims are expressed.

## Claims

1. A guide extension catheter (214, 314, 414), comprising:
a proximal member (216, 316, 416) having a proximal outer diameter, wherein the proximal member (216, 316, 416) takes the form of a tether wire;
a removable stiffening member (246, 346, 446) disposed adjacent to the proximal member (216, 316, 416), wherein the stiffening member (216, 316, 416) includes a tube; and
a distal sheath member (226, 326, 426) attached to the proximal member (216, 316, 416), the distal sheath member (226, 326, 426) having a distal outer diameter greater than the proximal outer diameter.

2. The guide extension catheter of claim 1, wherein the tether wire is disposed within a lumen of the tube.

3. The guide extension catheter of claim 1, wherein the tether wire is disposed along an outer surface of the tube.

## Patentansprüche

1. Führungsverlängerungskatheter (214, 314, 414), der aufweist:
ein proximales Element (216, 316, 416) mit einem proximal Außendurchmesser, wobei das proximale Element (216, 316, 416) die Form eines Haltedrahts annimmt;
ein entfernbares Versteifungselement (246, 346, 446), das benachbart zum proximalen Element (216, 316, 416) angeordnet ist, wobei das Versteifungselement (246, 346, 446) eine Röhre aufweist; und
ein distales Hüllenelement (226, 326, 426), das am proximalen Element (216, 316, 416) angebracht ist, wobei das distale Hüllenelement (226, 326, 426) einen distalen Außendurchmesser aufweist, der größer als der proximale Außendurchmesser ist.

2. Führungsverlängerungskatheter nach Anspruch 1, wobei der Haltedraht innerhalb eines Lumens der Röhre angeordnet ist.

3. Führungsverlängerungskatheter nach Anspruch 1, wobei der Haltedraht längs einer Außenfläche der Röhre angeordnet ist.

## Revendications

1. Cathéter d'extension de guidage (214, 314, 414), comprenant :
un élément proximal (216, 316, 416) présentant un diamètre externe proximal, dans lequel l'élément proximal (216, 316, 416) prend la forme d'un câble d'ancrage ;
un élément raidisseur détachable (246, 346, 446) disposé à côté de l'élément proximal (216, 316, 416), dans lequel l'élément raidisseur (246, 346, 446) inclut un tube ; et
un élément de gaine distal (226, 326, 426) fixé à l'élément proximal (216, 316, 416), l'élément de gaine distal (226, 326, 426) présentant un diamètre externe distal supérieur au diamètre externe proximal.

2. Cathéter d'extension de guidage selon la revendication 1, dans lequel le câble d'ancrage est disposé à l'intérieur d'une lumière du tube.

3. Cathéter d'extension de guidage selon la revendication 1, dans lequel le câble d'ancrage est disposé le long d'une surface externe du tube.
